(19) **Europäisches Patentamt European Patent Office Office européen des brevets**

(11) **EP 4 538 230 A1**

(12) **EUROPEAN PATENT APPLICATION**
published in accordance with Art. 153(4) EPC

(43) Date of publication:
**16.04.2025 Bulletin 2025/16**

(21) Application number: **23823863.8**

(22) Date of filing: **09.06.2023**

(51) International Patent Classification (IPC):
**C01B 33/193** *(2006.01)*      **A61Q 1/12** *(2006.01)*
**A61K 8/25** *(2006.01)*

(52) Cooperative Patent Classification (CPC):
**A61K 8/25; A61Q 1/12; C01B 33/193**

(86) International application number:
**PCT/JP2023/021631**

(87) International publication number:
**WO 2023/243573 (21.12.2023 Gazette 2023/51)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **13.06.2022   JP 2022095198**
**19.01.2023   JP 2023006655**

(71) Applicants:
• **AGC INC.**
**Chiyoda-ku,**
**Tokyo 1008405 (JP)**
• **AGC Si-Tech Co., Ltd.**
**Fukuoka 808-0027 (JP)**

(72) Inventors:
• **ARIMITSU Shinnosuke**
**Tokyo 100-8405 (JP)**
• **KATAYAMA Hajime**
**Tokyo 100-8405 (JP)**
• **KONDO Masashi**
**Tokyo 100-8405 (JP)**
• **FUKUMOTO Kohta**
**Tokyo 100-8405 (JP)**
• **KAMO Hiromichi**
**Tokyo 100-8405 (JP)**
• **YAMAZUMI Daisuke**
**Kitakyushu-shi, Fukuoka 808-0027 (JP)**

(74) Representative: **Müller-Boré & Partner**
**Patentanwälte PartG mbB**
**Friedenheimer Brücke 21**
**80639 München (DE)**

(54) **SILICA POWDER FOR COSMETIC PREPARATIONS, AND COSMETIC PREPARATION**

(57)    The present invention provides a silica powder for cosmetic preparations, the silica powder being composed of amorphous silica particles, wherein the average circularity of the silica particles is 0.75 or more. This silica powder satisfies at least one of the following conditions: (1) the agglomeration degree is 60% or more; (2) the angle of difference between the angle of repose and the angle of fall is 15 degrees or less; and (3) the degree of dispersity is 50% or less.

**EP 4 538 230 A1**

**Description**

TECHNICAL FIELD

The present invention relates to a silica powder for a cosmetic material and a cosmetic material.

BACKGROUND ART

For adjusting a feeling in use and blend with skin, a cosmetic material is mixed with extremely fine resin particles (microplastics) such as methacrylic acid methyl ester (PMMA) particles, nylon particles, silicone particles, and polyurethane particles.

Since microplastics are extremely fine, they are difficult to remove at sewage treatment plants, and there are concerns that they may flow into rivers, oceans, ponds, and the like, causing an impact on the marine ecosystem. In addition, microplastics may accumulate in seafood, which may have an impact on a human body. Therefore, there is a demand for development of materials that are more friendly to the environment and human bodies as alternatives to resin particles used in cosmetic materials.

Examples of a material that is friendly to the environment and human bodies include silica particles containing silica ($SiO_2$) as a main component, and for example, Patent Literature 1 provides a cosmetic product mixed with 3 wt% to 70 wt% of monodispersed non-porous spherical silica obtained by dispersing a porous silica gel in a high-temperature gas and firing the gel to make it non-porous.

CITATION LIST

PATENT LITERATURE

Patent Literature 1: JP2921058B

SUMMARY OF INVENTION

TECHNICAL PROBLEM

The silica powder including silica particles has a strongly smooth and dry feel, but it is difficult to obtain a moist feel that can be obtained with a powder including soft particles such as nylon particles or urethane particles.

Therefore, an object of the present invention is to provide a silica powder for use in a cosmetic material which has a new feel different from silica powders in the related art and can impart a moist feel when mixed in a cosmetic material.

SOLUTION TO PROBLEM

As a result of diligent studies, the present inventors have found that the above problems can be solved by using a silica powder that satisfies certain conditions, and have completed the present invention based on this finding.

That is, the present invention relates to the following.

<1> A silica powder for a cosmetic material, including:

amorphous silica particles, in which
the silica particles have an average circularity of 0.75 or more, and
the silica powder satisfies at least one of the following conditions (1) to (3),

(1) a degree of aggregation of 60% or more,
(2) an angle of difference between an angle of repose and an angle of rupture of 15 degrees or less, or
(3) a degree of dispersion of 50% or less.

<2> The silica powder for a cosmetic material according to <1>, having a stress relaxation rate of 18% or less when a load of 50 N is applied.
<3> The silica powder for a cosmetic material according to <1> or <2>, having a shearing cohesion of 2.0 kPa or more when a load of 50 N is applied.
<4> The silica powder for a cosmetic material according to any one of <1> to <3>, having a specific surface area of less than 5 $m^2$/g.

<5> The silica powder for a cosmetic material according to any one of <1> to <4>, in which

the silica powder has a 50% particle diameter ($D_{50}$) in a volume-based cumulative particle size distribution of 0.5 µm to 100 µm, and

the silica powder has a ratio ($D_{90}/D_{10}$), which is a ratio of a 90% particle diameter ($D_{90}$) in the volume-based cumulative particle size distribution to a 10% particle diameter ($D_{10}$) in the volume-based cumulative particle size distribution, of 1.0 to 5.0.

<6> A cosmetic material including the silica powder for a cosmetic material according to any one of <1> to <5>.

<7> The cosmetic material according to <6>, further including one or more types of silica powders other than the silica powder for a cosmetic material.

ADVANTAGEOUS EFFECTS OF INVENTION

According to the present invention, it is possible to provide a silica powder for a cosmetic material which can impart a moist feel when mixed in a cosmetic material. Furthermore, it is possible to provide a cosmetic material including such a silica powder for a cosmetic material.

DESCRIPTION OF EMBODIMENTS

The present invention will be described below, and the present invention is not limited by exemplifications in the following description. Note that in the present description, an expression "to" used to express a numerical range includes numerical values before and after it as a lower limit value and an upper limit value of the range, respectively.

<Silica Powder for Cosmetic Material>

A silica powder for a cosmetic material of the present embodiment is a silica powder including amorphous silica particles, in which the silica particles have an average circularity of 0.75 or more, and the silica powder satisfies at least one of the following conditions (1) to (3).

(1) a degree of aggregation of 60% or more
(2) an angle of difference between an angle of repose and an angle of rupture of 15 degrees or less
(3) a degree of dispersion of 50% or less

Here, in the present description, the term "powder" is used to mean an aggregate of individual particles. That is, the silica powder means an aggregate of silica particles.

The silica particles constituting the silica powder for a cosmetic material (hereinafter simply referred to as silica powder) of the present embodiment are amorphous. Since the silica particles are amorphous, it is possibles to provide the silica powder that can be used in cosmetic applications. Note that in the present description, the term "amorphous" refers to a diffraction peak indicating a crystal not being observed by using powder X-ray diffraction.

The silica particles constituting the silica powder of the present embodiment have an average circularity of 0.75 or more. When the average circularity is 0.75 or more, a shape of the particles becomes close to a sphere, so that a cosmetic material including the silica powder can be easily spread on skin when applied thereto. The average circularity is preferably 0.80 or more, more preferably 0.85 or more, still more preferably 0.90 or more, and particularly preferably 0.95 or more. An upper limit of the average circularity is not particularly limited, and is most preferably 1. The average circularity is, for example, 0.75 to 1.

Note that the circularity can be calculated by obtaining an area and a perimeter of a particle with an image obtained by a particle image analyzer (for example, Morphologi 4 manufactured by Malvern Panalytical Ltd) by using image analysis software attached to the above analyzer and applying the results to the following equation. The average circularity is an average value of 20 particles.

$$\text{Circularity} = \text{perimeter of circle with equal projected area}/\text{perimeter of particle}$$

Perimeter of circle with equal projected area: observing a particle from directly above, obtaining an area of a shadow of the particle reflected on a plane below, and calculating a circle having an area equal to this area, so as to obtain a length of an outline of the circle

Perimeter of particle: observing the particle from directly above to obtain a length of an outline of the shadow of the particle reflected on the plane below

The silica powder of the present embodiment satisfies at least one of the following conditions (1) to (3).

(1) a degree of aggregation of 60% or more
(2) an angle of difference between an angle of repose and an angle of rupture of 15 degrees or less
(3) a degree of dispersion of 50% or less

Since the silica powder of the present embodiment satisfies at least one of the above conditions (1) to (3), a soft and moist feel is imparted, and therefore an excellent moist feel when mixed in a cosmetic material can also be imparted.

Here, when the degree of aggregation of the silica powder of the present embodiment is 60% or more (condition (1)), an interaction between the silica particles is large and adhesion between the particles is improved, resulting in a soft and moist feel when spread on skin. The degree of aggregation is preferably 65% or more, more preferably 70% or more, still more preferably 75% or more, and particularly preferably 80% or more. An upper limit of the degree of aggregation is not particularly limited, and the degree of aggregation is preferably 100%. The degree of aggregation is, for example, 60% to 100%.

The degree of aggregation can be measured by a powder tester (for example, model PT-X, manufactured by Hosokawa Micron Corporation). Specifically, a sieve with an opening size of 250 $\mu$m, a sieve with an opening size of 150 $\mu$m, and a sieve with an opening size of 75 $\mu$m are set on a vibration table of the powder tester from top to bottom, 2.0 g of powder is placed on a top sieve (with an opening size of 250 $\mu$m) and vibrated with a vibration amplitude of 1.0 mm for 90 seconds, a mass of powder remaining on each sieve is measured, and the degree of aggregation is calculated based on the following formula.

$$\text{Degree of aggregation (\%)} = \{(A + B \times 3/5 + C \times 1/5)/2\} \times 100$$

A: mass of powder on top sieve, B: mass of powder on middle sieve, C: mass of powder on bottom sieve

When the angle of difference between the angle of repose and the angle of rupture of the silica powder of the present embodiment is 15 degrees or less (condition (2)), the interaction between the silica particles is large, so that the adhesion is improved, and a soft and moist feel is obtained. The angle of difference is preferably 14 degrees or less, more preferably 13 degrees or less, still more preferably 12 degrees or less, particularly preferably 11 degrees or less, and most preferably 10 degrees or less. The angle of difference is preferably 0 degree or more, more preferably 0.5 degrees or more, and still more preferably 1 degree or more. The angle of difference is, for example, 0 degree to 15 degrees.

Here, the angle of repose and the angle of rupture will be described.

The angle of repose is an angle between a horizontal plane and a slope of the cone-shaped mountain that is formed when a powder is dropped from a funnel of a certain height and remains stable without spontaneously collapsing.

The angle of repose of the silica powder of the present embodiment is preferably 20 degrees or more. By having an angle of repose of 20 degrees or more, the silica powder can be spread evenly when applied onto skin. The angle of repose is more preferably 22 degrees or more, still more preferably 25 degrees or more, and particularly preferably 30 degrees or more. From the viewpoint of ease of spreading, the angle of repose is preferably 80 degrees or less, more preferably 75 degrees or less, and still more preferably 70 degrees or less. The angle of repose is preferably 20 degrees to 80 degrees.

The angle of rupture refers to an angle between a generating line of the cone and the horizontal plane when a specified impact is applied to the cone-shaped mountain formed in the measurement of the angle of repose to cause a part of the cone to collapse.

The angle of rupture of the silica powder of the present embodiment is preferably 20 degrees or more. When the angle of rupture is 20 degrees or more, the adhesion between the silica particles is very high, and an excellent moist feel is obtained. The angle of rupture is more preferably 22 degrees or more, still more preferably 25 degrees or more, and particularly preferably 30 degrees or more. The angle of rupture is preferably 70 degrees or less, more preferably 65 degrees or less, and still more preferably 60 degrees or less. The angle of rupture is preferably 20 degrees to 70 degrees.

The angle of repose and the angle of rupture can be measured using a powder tester (for example, model PT-X, manufactured by Hosokawa Micron Corporation).

Specifically, a measuring platform with a diameter of 80 mm is placed horizontally, and a sieve with an opening size of 1.7 mm and a funnel (for example, made of stainless steel, upper opening diameter: 70 mm, lower opening diameter: 7 mm, inclination angle: 63 degrees, height from the measuring platform to the lower opening of the funnel: 7.5 cm) are placed above the measuring platform. To the sieve, 250 mL of the powder is gently placed, and then vibrated with an amplitude of 1.5 mm to allow the powder to fall through the sieve and funnel onto the measuring platform, depositing the powder in a cone shape. Next, the cone shape is photographed from directly beside, and an angle between the generating line and the horizontal plane of the cone is measured once, which is taken as the angle of repose. The angle of rupture is measured by applying the same impact three times to the powder for which the angle of repose is measured using an automatic shocker attached to the device, then photographing the conical shape of the sample from directly beside, and measuring an angle between the generating line and the horizontal plane of the cone once, which is taken as the angle of rupture.

When the silica powder of the present embodiment has a degree of dispersion of 50% or less (condition (3)), the interaction between the silica particles is increased, the adhesion is improved, and a moist feel can be obtained. The degree of dispersion is preferably 45% or less, more preferably 40% or less, still more preferably 35% or less, and particularly preferably 30% or less. The degree of dispersion of the silica powder is preferably 0% or more, and more preferably 1% or more. The degree of dispersion is, for example, 0% to 50%.

Here, the degree of dispersion (%) is, when a certain amount of silica powder is dropped onto a measuring platform, a ratio of a mass of the powder that is scattered without dropping onto the measuring platform to a mass of the powder before dropping onto the measuring platform. The degree of dispersion (%) can be measured using a powder tester (for example, model PT-X, manufactured by Hosokawa Micron Corporation). Specifically, 10 g of a powder is placed on a top of a degree of dispersion measuring unit of the powder tester, and the powder is allowed to drop onto a watch glass with a diameter of 100 mm placed 60 cm below the degree of dispersion measuring unit. A mass of the powder accumulated on the watch glass is weighed, and the degree of dispersion is calculated according to the following formula.

Degree of dispersion (%) = {(mass (g) of powder before dropping - mass (g) of powder accumulated on watch glass)/ mass (g) of powder before dropping} $\times$ 100

The silica powder of the present embodiment may satisfy only one of the above conditions (1) to (3), any two of the above conditions (1) to (3), or all of the above conditions (1) to (3). Among these, from the viewpoints of improving the adhesion between silica particles and further enhancing the moist feel, it is preferable that all of the above conditions (1) to (3) are satisfied.

Furthermore, when only one or two of the above conditions (1) to (3) are satisfied, the effect of improving the moist feel is most exhibited, and therefore it is particularly preferable that the condition (1) is satisfied.

(50% particle diameter ($D_{50}$))

The 50% particle diameter ($D_{50}$) in the volume-based cumulative particle size distribution of the silica powder is preferably 0.5 $\mu$m to 100 $\mu$m. When the 50% particle diameter ($D_{50}$) of the silica powder of the present embodiment is 0.5 $\mu$m or more, secondary aggregation of the silica particles can be prevented, and good dispersion in a cosmetic material can be easily obtained. $D_{50}$ is more preferably 0.7 $\mu$m or more, still more preferably 0.9 $\mu$m or more, and particularly preferably 1.0 $\mu$m or more.

When $D_{50}$ is 100 $\mu$m or less, the silica particles are not too large, and the feeling in use when mixed in a cosmetic material is improved. $D_{50}$ is more preferably 50 $\mu$m or less, still more preferably 20 $\mu$m or less, and particularly preferably 15 $\mu$m or less.

A ratio ($D_{90}/D_{10}$) of a 90% particle diameter ($D_{90}$) in the volume-based cumulative particle size distribution to a 10% particle diameter ($D_{10}$) in the volume-based cumulative particle size distribution of the silica powder is preferably 1.0 to 5.0. When $D_{90}/D_{10}$ of the silica powder of the present embodiment is within the above range, the amount of coarse particles is reduced and an increase in the specific surface area due to fine particles is prevented, so that a good feel can be obtained when applied to skin. $D_{90}/D_{10}$ is more preferably 4.5 or less, still more preferably 4.0 or less, and particularly preferably 3.5 or less. $D_{90}/D_{10}$ is more preferably 1.1 or more, still more preferably 1.2 or more, and particularly preferably 1.5 or more.

Note that the 10% particle diameter ($D_{10}$), 50% particle diameter ($D_{50}$) and 90% particle diameter ($D_{90}$) in the volume-based cumulative particle size distribution can be calculated by a particle size distribution measuring device (for example, Multisizer 4e manufactured by Beckman Coulter, Inc.) using an electrical detection method.

(Shearing cohesion)

The silica powder of the present embodiment preferably has a shearing cohesion of 2.0 kPa or more when a load of 50 N is applied thereto.

The shearing cohesion indicates adhesion between the silica particles, and the greater the shearing cohesion, the stronger the adhesion, and the moist feel is improved when the silica powder is mixed in a cosmetic material. When the shearing cohesion is 2.0 kPa or more, a strong moist feel can be obtained. The shearing cohesion of the silica powder is more preferably 2.2 kPa or more, still more preferably 2.4 kPa or more, and particularly preferably 2.6 kPa or more. The shearing cohesion is preferably 5.0 kPa or less, more preferably 4.5 kPa or less, and still more preferably 4.0 kPa or less. That is, the shearing cohesion of the silica powder is preferably in a range of 2.0 kPa to 5.0 kPa.

The shearing cohesion is measured using a powder bed shear force measuring device (for example, model NS-S500, manufactured by Nano Seeds Corporation) in accordance with JIS Z 8835:2016 under the following conditions, and a yield locus (YL) is obtained by plotting a shear stress (y-axis) corresponding to each normal stress (x-axis). The shearing cohesion is obtained from an intersection of YL and the y-axis.

Conditions: cell inner diameter 43 mm, indentation gap 0.2 mm, indentation speed 0.2 mm/sec, stress relaxation time 100 sec, shear rate 10 μm/sec, normal load 50 N

(Stress Relaxation Rate)

The stress relaxation rate is a degree of rearrangement of the silica particles when the silica powder is compressed under a load. The smaller the stress relaxation rate, the greater the interaction between the silica particles. In this way, the adhesion between the silica particles is improved, and a moist feel can be obtained.

The silica powder of the present embodiment preferably has a stress relaxation rate of 18% or less when a load of 50 N is applied thereto. When the stress relaxation rate is 18% or less, a sufficiently moist feel can be obtained. The stress relaxation rate of the silica powder is more preferably 17% or less, still more preferably 16% or less, and particularly preferably 15% or less. A lower limit of the stress relaxation rate is not particularly limited, and the stress relaxation rate is preferably 1% or more, and more preferably 3% or more. That is, the stress relaxation rate of the silica powder is preferably in a range of 1% to 18%.

The stress relaxation rate can be measured by a powder bed shear force measuring device (for example, model NS-S500, manufactured by Nano Seeds Corporation).

Specifically, a normal load applied to a powder bed is set to 50 N, and the stress relaxation rate (%) corresponding to the normal stress at the start of shearing can be calculated using the following formula from a maximum normal load a on an upper surface of the powder bed during pre-consolidation and a normal load b on the upper surface of the powder bed at 100 seconds after stress relaxation.

$$\text{Stress relaxation rate (\%)} = (1 - b/a) \times 100$$

(Static Friction Coefficient)

The silica powder of the present embodiment preferably has a static friction coefficient of 0.50 or more. When the static friction coefficient is 0.50 or more, both resistance when starting to apply to skin and a moist feel can be achieved. The static friction coefficient is more preferably 0.51 or more, still more preferably 0.53 or more, and particularly preferably 0.54 or more. From the viewpoint of reducing resistance at the start of application, the static friction coefficient is preferably 1.00 or less, more preferably 0.95 or less, and still more preferably 0.90 or less. That is, the static friction coefficient of the silica powder is preferably in a range of 0.50 to 1.00.

Note that the dynamic friction coefficient can be measured using a static and dynamic friction measuring device (for example, "TL201 Tt" manufactured by Trinity-Lab. Inc.). Specifically, a urethane artificial finger is used as a contactor, and a load is set to 30 gf, a scanning distance is set to 40 mm, and a scanning speed is set to 10 mm/sec, and artificial leather "sapurare" (for example, manufactured by IDEATEX JAPAN Co., Ltd.) is used as a coating substrate, and the friction coefficient is measured with a coating amount of the silica powder being 0.8 μL/cm$^2$ in terms of a bulk volume per unit area. A maximum value of obtained friction coefficients in a range of 0 msec to 1,000 msec is regarded as the static friction coefficient.

(Dynamic Friction Coefficient)

The silica powder of the present embodiment preferably has a dynamic friction coefficient of 0.30 or more. When the dynamic friction coefficient is 0.30 or more, the silica powder can be spread on skin while maintaining a moist feel. The dynamic friction coefficient is more preferably 0.33 or more, still more preferably 0.35 or more, and particularly preferably 0.36 or more. From the viewpoint of ease of spreading, the dynamic friction coefficient is preferably 0.80 or less, more preferably 0.75 or less, and still more preferably 0.70 or less. That is, the dynamic friction coefficient of the silica powder is preferably in a range of 0.30 to 0.80.

The static friction coefficient can be measured by the above-mentioned static and dynamic friction measuring device. Specifically, the friction coefficient is measured in the same manner as above, and an average value of the obtained friction coefficients in a range of 1,000 msec to 4,000 msec is regarded as the dynamic friction coefficient.

(Aerated Bulk Density)

The silica powder of the present embodiment preferably has an aerated bulk density of 0.45 g/cm$^3$ or more and 0.85 g/cm$^3$ or less. The aerated bulk density refers to a bulk density measured when the powder is gently packed into a container. The powder gently packed is in a state where many gaps exist between the particles. When the aerated bulk density is within the above range, the interaction between the silica particles is strong, resulting in many gaps and a large rate of change corresponding to a load, so that a moist feel can be easily obtained. The aerated bulk density is more preferably 0.47 g/cm$^3$ or more and 0.83 g/cm$^3$ or less, still more preferably 0.49 g/cm$^3$ or more and 0.81 g/cm$^3$ or less, and particularly preferably 0.51 g/cm$^3$ or more and 0.79 g/cm$^3$ or less.

(Packed Bulk Density)

The silica powder of the present embodiment has a packed bulk density of 1.0 g/cm$^3$ or more and 1.5 g/cm$^3$ or less. The packed bulk density is a bulk density measured after a container gently filled with a powder is vibrated to fill gaps between the particles and then refilled with a powder. When the packed bulk density is within the above range, the particles can be said to be compacted. The packed bulk density is more preferably 1.02 g/cm$^3$ or more and 1.40 g/cm$^3$ or less, still more preferably 1.04 g/cm$^3$ or more and 1.35 g/cm$^3$ or less, and particularly preferably 1.05 g/cm$^3$ or more and 1.30 g/cm$^3$ or less.

Note that the aerated bulk density and the packed bulk density can be measured by a powder tester (for example, model PT-X, manufactured by Hosokawa Micron Corporation).

Specifically, the silica powder is poured gently into a special cup, and the special cup is filled with the silica powder until overflowing from an opening thereof, and then the powder that overflows from the special cup is scraped off from a surface with an attached blade, and a mass including the cup is measured. A mass of the silica powder is calculated by subtracting a mass of the cup, which is measured in advance, from the above value, and the mass is divided by an internal volume of the cup to obtain the aerated bulk density.

Next, a cap is put on the special cup, and then the cup is placed in a tapping holder of the powder tester, and the cup is then further filled with the silica powder, and a cap cover is attached on the cap. The cup is then tapped 180 times. After tapping is completed, the cap and the cap cover are removed, followed by scraping off the surface of the special cup with the attached blade, and a mass including the cup is measured. A mass of the silica powder is calculated by subtracting the mass of the cup, which is measured in advance, from the above value, and the mass is divided by the internal volume of the cup to obtain the packed bulk density.

(Degree of Compression)

A degree of compression is a rate of reduction in volume of the powder from the state where no load is applied to the powder to the state where a set load is applied to the powder.

The silica powder of the present embodiment preferably has a degree of compression of 37% or more. When the degree of compression is 37% or more, the silica powder is compacted in response to a load when being applied to skin, so that a moist feel can be provided. The degree of compression is more preferably 38% or more, further preferably 39% or more, and particularly preferably 39.5% or more. The degree of compression is preferably 70% or less, more preferably 65% or less, still more preferably 60% or less, and particularly preferably 55% or less. That is, the degree of compression of the silica powder is preferably in a range of 37% to 70%.

The degree of compression is calculated by the following formula using the above aerated bulk density value and packed bulk density value.

$$\text{Degree of compression (\%)} = \{(\text{packed bulk density - aerated bulk density})/\text{packed bulk density}\} \times 100$$

(Specific Surface Area)

The silica powder of the present embodiment preferably has a specific surface area of less than 5 m$^2$/g as determined based on a nitrogen adsorption method. When the specific surface area of the silica powder is less than 5 m$^2$/g, the number of silanol groups in the silica particles is reduced, improving the adhesion between the silica particles, thereby improving the moist feel when the silica powder is mixed in a cosmetic material.

The smaller the specific surface area of the silica powder, the better the moist feel can be imparted, and therefore, the specific surface area is more preferably 4.5 m$^2$/g or less, still more preferably 4 m$^2$/g or less, particularly preferably 3 m$^2$/g or less, and most preferably 2 m$^2$/g or less. A lower limit thereof is not particularly limited, and the specific surface area is preferably 0.1 m$^2$/g or more. That is, the specific surface area of the silica powder is preferably 0.1 m$^2$/g or more and less

than 5 $m^2/g$.

Note that the specific surface area can be calculated using the BET theory after obtaining an adsorption isotherm by the nitrogen adsorption method.

(Pore Volume)

The pore volume of the silica powder of the present embodiment is preferably less than 0.1 $cm^3/g$. Here, the pore volume refers to a pore volume derived from pores with a pore diameter of 1 nm to 100 nm. Since the specific surface area can be reduced by dissolving the silica component through firing and making the silica component non-porous, the pore volume is preferably less than 0.1 $cm^3/g$.

The pore volume of the silica powder is more preferably 0.08 $cm^3/g$ or less, still more preferably 0.06 $cm^3/g$ or less, and particularly preferably 0.04 $cm^3/g$ or less. A lower limit value of the pore volume of the silica powder is not particularly limited, and the pore volume is preferably 0 $cm^3/g$ or more, more preferably 0.001 $cm^3/g$ or more, and still more preferably 0.005 $cm^3/g$ or more. That is, the pore volume of the silica powder is preferably 0 $cm^3/g$ or more and 0.1 $cm^3/g$ or less.

Note that the pore volume can be calculated using the BJH method by a nitrogen adsorption method.

(Particle Strength)

The silica powder of the present embodiment preferably has a particle strength of 10 MPa or more. When the particle strength is 10 MPa or more, the particles constituting the silica powder have a strength that makes the powder less likely to break, resulting in a good feel when mixed in a cosmetic material.

The particle strength of the silica powder is more preferably 15 MPa or more, still more preferably 20 MPa or more, and particularly preferably 25 MPa or more, and the upper limit is not particularly limited.

Note that the particle strength of the silica powder can be measured using a micro-compression tester (for example, MCT-510, manufactured by Shimadzu Corporation). In the present embodiment, the particle strength is determined by measuring compressive strength of 40 silica particles and averaging the results.

(Sodium Concentration)

The silica powder of the present embodiment may include sodium at a concentration of 1 ppm to 300 ppm. By including sodium at a concentration within the above range, it becomes possible to perform firing at high temperatures while preventing crystallization. The sodium concentration is preferably 2 ppm or more, more preferably 3 ppm or more, and still more preferably 10 ppm or more. The sodium concentration is preferably 200 ppm or less, more preferably less than 200 ppm, still more preferably 150 ppm or less, particularly preferably 100 ppm or less, and most preferably 50 ppm or less.

The silica particles constituting the silica powder of the present embodiment may include an impurity element other than sodium as long as the effect of the present invention is not impaired. Examples of the other impurity elements include Mg, Ca, Al, Fe, and Ti.

A concentration of each of the other impurity elements is preferably 500 ppm or less, more preferably 300 ppm or less, and still more preferably 100 ppm or less.

The concentration of sodium and the other impurity elements can be measured by inductively coupled plasma (ICP) emission spectrometry after adding perchloric acid and hydrofluoric acid to the silica powder, igniting the mixture, and removing silicon which is the main component.

(Oil Absorption Value)

The silica particles constituting the silica powder of the present embodiment are preferably non-porous particles. In a case of porous particles, an oil absorption value is high, and the adhesion between the silica particles is reduced, which may result in a reduction in the moist feel. Specifically, the oil absorption value of the silica powder is preferably 100 ml/100 g or less, more preferably 70 ml/100 g or less, and most preferably 50 ml/100 g or less. A lower limit value thereof is not particularly limited, but it is substantially difficult to set the oil absorption value to 20 ml/100 g or less.

Regarding measurement of the oil absorption value, it is preferable to use boiled linseed oil in accordance with JIS K5101-13-2:2004.

<Method for Producing Silica Powder for Cosmetic Material>

The silica powder of the present embodiment can be obtained, for example, by forming a spherical silica precursor and subjecting the silica precursor to a heat treatment.

The silica precursor may be obtained by production, or a commercially available product may be used. Examples of a method for producing the silica precursor include a wet method and a granulation method.

The wet method refers to a method including a step of obtaining a raw material of the spherical silica particles by using a liquid as a silica source and gelling the liquid. The granulation method is a method in which silica fine particles are molded into spheres using a binder or the like. Between the two methods, by using the wet method that can control the particle size distribution and produce spherical particles, spherical silica particles can be formed, and therefore, there is no need to adjust the shape of the particles by crushing or the like, and as a result, particles with a small specific surface area are obtained. In the wet method, the particles having a particle diameter significantly smaller than an average particle diameter are hardly generated, and the specific surface area tends to be smaller after firing. In the wet method, the amount of the impurity element such as titanium can be adjusted by controlling impurities of the silica source, and the above-described impurity element can be uniformly dispersed in the particles.

Examples of the wet method include a spraying method and an emulsion gelling method. In the emulsion gelling method, for example, a dispersed phase containing a silica precursor and a continuous phase are emulsified, and the obtained emulsion is gelled to obtain a spherical silica precursor. As an emulsification method, it is preferable to prepare an emulsion by supplying the dispersed phase containing the silica precursor to the continuous phase through fine pores or a porous film. Accordingly, an emulsion having a uniform liquid droplet diameter is prepared, and as a result, a silica powder including silica particles having a uniform particle diameter is obtained. Here, the particle diameter of the silica particles can be adjusted by changing the stirring speed during emulsification.

Such an emulsification method may be a micromixer method or a film emulsification method. For example, the micromixer method is disclosed in WO2013/062105A.

The silica precursor obtained by the wet method is preferably porous. Since the silica precursor is porous, when it is fired to obtain the silica particles, it is easier to obtain silica particles with a controlled shape and powder particle size distribution as compared with a case where silica particles are produced by firing a pulverized non-porous raw material. When the porous silica precursor is heated, primary particles of several nm to several tens of nm that constitute an internal pore structure are fused, and thus the surface area decreases, and the pores become filled. It is also known that the presence of sodium decreases a fusing point.

Here, the silica precursor is said to be porous if pores are uniformly distributed in the silica precursor.

The pore volume of the silica precursor obtained by the wet method is desirably 0.05 ml/g to 2.2 ml/g. In the case where the pore volume of the silica precursor is 0.05 ml/g or more, the silica particles sufficiently shrink during firing, and the specific surface area can be reduced. In the case where the pore volume of the silica precursor is 2.2 ml/g or less, it is possible to prevent an excessive increase in prepared bulk density before firing, and to improve productivity. The pore volume of the silica precursor is more preferably 0.1 ml/g to 2.2 ml/g, more preferably 0.3 ml/g to 2.2 ml/g, still more preferably 0.3 ml/g to 1.8 ml/g, particularly preferably 0.6 ml/g to 1.8 ml/g, and most preferably 0.7 ml/g to 1.5 ml/g.

The pore volume can be measured by the same method as described above.

An ignition loss of the silica precursor obtained by the wet method is desirably 5.0 mass% to 15.0 mass%. The ignition loss is a sum of a mass of adhering water adhered to the silica precursor and a mass of water generated from condensation of the silanol group contained in the silica precursor, and the silica precursor has an appropriate silanol group, so that the condensation progresses during firing, and the silanol group easily decreases. As the silanol groups decrease, the silica particles become charged and the interaction between the silica particles increases. When the ignition loss is too large, a yield during firing may be lowered and productivity may deteriorate, and therefore, the ignition loss of the silica precursor is preferably 15.0 mass% or less, more preferably 13.0 mass% or less, and most preferably 12.0 mass% or less. When the ignition loss is too small, the silanol group is likely to remain during firing, and therefore, the ignition loss of the silica precursor is preferably 5.0 mass% or more, more preferably 6.0 mass% or more, and most preferably 7.0 mass% or more.

Here, the ignition loss is determined in accordance with JIS K 0067 (1992) as a mass loss when 1 g of the silica precursor is heated and dried at 850°C for 0.5 hours.

An average pore diameter of the silica precursor obtained by the wet method is preferably 1.0 nm to 50.0 nm. When the average pore diameter thereof is 1.0 nm or more, the silica particles can be made uniformly non-porous all the way to the inside. When the average pore diameter is 50.0 nm or less, the silica particles can be densified (made to have a lower specific surface area) by firing without any pores remaining, and thus, a moist feel can be obtained. The average pore diameter is more preferably 2.0 nm to 40.0 nm, still more preferably 3.0 nm to 30.0 nm, and particularly preferably 4.0 nm to 20.0 nm.

The average pore diameter is obtained by the BET method based on the nitrogen adsorption method using the specific surface area and pore distribution measuring device (for example, "BELSORP-mini II" manufactured by MicrotracBEL Corp., "TriStar II" manufactured by Micromeritics Instrument Corporation).

The silica precursor obtained by the wet method preferably has a mass loss rate of 10% or less when dried at 230°C for 12 hours. When the mass loss rate is 10% or less, sintering of the particles hardly occurs when the silica precursor is fired in a state where the particles are in contact with each other, and the spherical silica particles are easily obtained.

The mass loss rate is more preferably 9% or less, still more preferably 8% or less, and particularly preferably 6% or less, and since it is desirable that the mass does not change even after drying at 230°C for 12 hours, a lower limit thereof is not particularly limited.

When a water content of the obtained silica precursor is large and the mass loss rate when the silica precursor is dried at 230°C for 12 hours is more than 10%, the silica precursor is preferably dried so that the mass loss rate becomes 10% or less. Examples of drying means include a spray dryer, static drying in a dryer, and ventilation treatment with dry air.

A method for performing a heat treatment on the silica precursor is not particularly limited, and examples thereof include heat treatment with leaving to stand, heat treatment by a rotary furnace, and heat treatment by spray combustion. For the heat treatment with leaving to stand, a stationary electric furnace, a roller hearth kiln, a continuous furnace classified as a tunnel furnace, or the like can be used. For the heat treatment by a rotary furnace, a horizontal rotary furnace (rotary kiln), a rotary tubular furnace, or the like can be used. Among these, from the viewpoint of uniformity of firing, it is preferable to fire by heat treatment using a continuous furnace or a rotary furnace. Here, by adjusting the temperature during the heat treatment, the degree of aggregation, the angle of repose, the angle of rupture, the degree of dispersion, the specific surface area, the shearing cohesion, the stress relaxation rate, the degree of compression, and the friction coefficients of the silica powder can be adjusted.

The heat treatment temperature is preferably 700°C or higher, more preferably 800°C or higher, still more preferably 900°C or higher, and particularly preferably 1000°C or higher. From the viewpoint of preventing crystallization, the heat treatment temperature is preferably 1600°C or lower, more preferably 1500°C or lower, and still more preferably 1400°C or lower. That is, the heat treatment temperature is preferably 700°C or higher and 1600°C or lower.

A firing time may be appropriately adjusted in accordance with a firing device to be used and the firing time, and the heat treatment is preferably performed, for example, for 0.5 hours to 50 hours, and more preferably 1 hour to 10 hours.

An atmosphere during the firing may be an atmosphere containing oxygen or an atmosphere containing no oxygen. In the case where the silica precursor is obtained by a wet method, an organic substance such as an emulsion is often used, and thus, the organic substance often remains in the silica precursor. When a silica precursor containing an organic substance is fired, the organic substance is carbonized under a condition with a small amount of oxygen, causing coloring. Accordingly, when the silica precursor contains an organic substance, firing is preferably performed in an oxygen-containing atmosphere, and more preferably in the air.

During firing, it is preferable to fire the silica precursor in a state where the particles are in contact with each other. When the particles are in contact with each other, firing can be performed with a small volume, and thus, as compared with, for example, a case where the silica precursor is dispersed in a gas and then fired, temperature irregularity and time irregularity during firing are smaller, and silica particles with consistent quality can be obtained. In a spray combustion method, in which a silica precursor is dispersed in a gas, even when a raw material powder is injected from a center of flame, a temperature of the gas being injected at the same time, a dispersion state of the raw material, a temperature difference due to different parts passing through the flame, a difference in time it takes to pass through the flame, and a time it takes to cool due to distribution of air flow (flow rate) inside the device, will lead to variations in firing conditions. By firing the silica precursor in the state where the particles are in contact with each other, the firing conditions for each silica precursor can be made uniform and a consistent quality can be maintained.

The silica particles may be weakly sintered together after firing, and thus, in that case, the silica particles may be crushed. In order to maintain the average circularity and specific surface area, crushing is preferably performed such that the average circularity of the particles does not fall below 0.75 so as not to impair effects of the present invention. It is preferable that the specific surface area is not increased by the crushing treatment. A large increase in the specific surface area by the crushing treatment means that some of the silica particles are crushed and fine damage occurs on the surface to generate a fine powder. An increase in the specific surface area is undesirable since it leads to a decrease in the moist feel when the silica powder is mixed in a cosmetic material.

The crushing can be performed using a crushing device such as a cyclone mill or a jet mill, or can also be performed using a vibrating sieve.

The silica particles obtained by firing may be subjected to a surface treatment with a silane coupling agent. By this step, silanol groups present on surfaces of the silica particles constituting the silica powder react with the silane coupling agent, so as to reduce the number of silanol groups on the surfaces, which can also improve dispersibility in oil.

Conditions for the surface treatment are not particularly limited, and general surface treatment conditions may be used, and a wet treatment method or a dry treatment method can be used. From the viewpoint of performing a uniform treatment, a wet treatment method is preferable.

Examples of the silane coupling agent used in the surface treatment include aminosilane coupling agents, epoxysilane coupling agents, mercaptosilane coupling agents, silane-based coupling agents, and organosilazane compounds. These may be used alone or in combination of two or more kinds thereof.

Specifically, examples of the surface treatment agent include an aminosilane coupling agent such as aminopropylmethoxysilane, aminopropyltriethoxysilane, ureidopropyltriethoxysilane, N-phenylaminopropyltrimethoxysilane, and N-2(aminoethyl)aminopropyltrimethoxysilane, an epoxysilane coupling agent such as glycidoxypropyltrimethoxysilane, glycidoxypropyltriethoxysilane, glycidoxypropylmethyldiethoxysilane, glycidylbutyltrimethoxysilane, and (3,4-epoxycyclohexyl)ethyltrimethoxysilane, a mercaptosilane coupling agent such as mercaptopropyltrimethoxysilane and mercaptopropyltriethoxysilane, a silane-based coupling agent such as methyltrimethoxysilane, vinyltrimethoxysilane, octadecyltrimethoxysilane, phenyltrimethoxysilane, methacroxypropyltrimethoxysilane, imidazolesilane, and triazinesilane, a fluorine-containing silane coupling agent such as $CF_3(CF_2)_7CH_2CH_2Si(OCH_3)_3$, $CF_3(CF_2)_7CH_2CH_2SiCl_3$, $CF_3(CF_2)_7CH_2CH_2Si(CH_3)(OCH_3)_2$, $CF_3(CF_2)_7CH_2CH_2Si(CH_3)Cl_2$, $CF_3(CF_2)_5CH_2CH_2SiCl_3$, $CF_3(CF_2)_5CH_2CH_2Si(OCH_3)_3$, $CF_3CH_2CH_2SiCl_3$, $CF_3CH_2CH_2Si(OCH_3)_3$, $C_8F_{17}SO_2N(C_3H_7)CH_2CH_2CH_2Si(OCH_3)_3$, $C_7F_{15}CONHCH_2CH_2CH_2Si(OCH_3)_3$, $C_8F_{17}CO_2CH_2CH_2CH_2Si(OCH_3)_3$, $C_8F_{17}$-O-$CF(CF_3)CF_2$-O-$C_3H_6SiCl_3$, and $C_3F_7$-O-$(CF(CF_3)CF_2$-O$)_2$-$CF(CF_3)CONH$-$(CH_2)_3Si(OCH_3)_3$, and an organosilazane compounds such as hexamethyldisilazane, hexaphenyldisilazane, trisilazane, cyclotrisilazane, and 1,1,3,3,5,5-hexamethylcyclotrisilazane.

A treatment amount of the silane coupling agent is preferably 0.01 parts by mass or more, more preferably 0.02 parts by mass or more, and still more preferably 0.10 parts by mass or more, and is preferably 5 parts by mass or less, and more preferably 2 parts by mass or less with respect to 100 parts by mass of the silica particles.

Examples of a method for treating with the silane coupling agent include a dry method in which the silane coupling agent is sprayed to the silica particles, and a wet method in which the silica particles are dispersed in a solvent and then the silane coupling agent is added to react with the particles.

Note that the fact that the surface of the silica particles is treated with the silane coupling agent can be confirmed by detecting a peak due to a substituent group of the silane coupling agent using IR. An adhesion amount of the silane coupling agent can be measured by an amount of carbon.

The silica powder is obtained as an aggregate of silica particles by the above method. Note that the silica particles obtained as described above are amorphous.

<Cosmetic Material>

A cosmetic material of the present embodiment includes the silica powder for a cosmetic material of the present embodiment. As described above, the silica powder for a cosmetic material of the present embodiment can impart a moist feel when mixed in a cosmetic material, and therefore the cosmetic material of the present embodiment exhibits a moist feel.

A content ratio of the silica powder for a cosmetic material of the present embodiment in the cosmetic material of the present embodiment can be appropriately adjusted.

The cosmetic material of the present embodiment may include other components that can be used in general cosmetic materials, in addition to the silica powder for a cosmetic material of the present embodiment. As other components, for example, water, oil components, water-miscible organic solvents, UV absorbers, antioxidants, preservatives/antibacterial agents, fragrances, surfactants, thickeners, inorganic powders, acids, alkalis, and sugars may be included. A content ratio of each of these other components in the cosmetic material according to the present embodiment can be adjusted appropriately depending on a purpose of use.

Furthermore, the cosmetic material according to the present embodiment may include, in addition to the silica powder of the present embodiment, one or more types of silica powders other than the silica powder of the present embodiment. By including a silica powder other than the silica powder of the present embodiment, a feel of the cosmetic material can be adjusted to be closer to that of resin, that is, to have a soft and moist feel.

A shape of the silica powder other than the silica powder of the present embodiment is preferably spherical, scaly, plate-like, balloon-like, or crushed, and more preferably spherical.

The cosmetic material according to the present embodiment may have any appropriate form depending on composition and purpose thereof. Examples of such a form include powder, liquid, cream, gel, and stick.

Examples of the cosmetic material according to the present embodiment include a makeup base, a foundation, a cosmetic cream, a sunscreen, a lotion, a milky lotion, a beauty serum, a facial pack, a facial cleanser, an eye color, a lipstick, a lip balm, a deodorant, an antiperspirant, and a water-based gel.

EXAMPLES

Hereinafter, the present invention will be described in detail with reference to Examples, but the present invention is not limited thereto. In the following description, common components employ the same substance.

Examples 1 to 6 are Working Examples, Examples 7 to 9 are Comparative Examples, and Example 10 is a Reference Example.

(Example 1)

As a dispersed phase, sodium silicate No. 3 (manufactured by AGC Si-Tech Co., Ltd., sodium silicate aqueous solution, $SiO_2/Na_2O$ (molar ratio) = 3, $SiO_2$ concentration = 24 mass%) was used in an amount of 30 g.

A mixture obtained by dissolving 0.7 mass% of sorbitan monooleate (manufactured by Sanyo Chemical Industries, Ltd., IONET S80) as a surfactant in n-decane (manufactured by Tosoh Corporation, HC-250, $C_{10}H_{22}$) in advance was used as a continuous phase in an amount of 150 g.

While stirring the continuous phase, which is the surfactant-containing n-decane, with a homogenizer (manufactured by MICROTEC CO., LTD., Physcotron, generator shaft NS-30UG/20P), the dispersed phase, which is the sodium silicate No. 3, was added and emulsified by stirring at 13,000 rpm for 5 minutes.

While stirring the obtained emulsion using a three-one motor, carbon dioxide gas was blown into the emulsion at a rate of 0.4 L/min for 20 minutes to precipitate silica.

Thereafter, while stirring an aqueous phase containing silica gel, which was obtained by removing an oil phase, at room temperature, sulfuric acid was added until a pH reached 2, and then the mixture was stirred in a warm bath at 80°C for 1 hour to remove residual decane. The slurry after removing the residual solvent was filtered and washed by pouring acidic demineralized water adjusted to pH 1 with sulfuric acid on the slurry at 40 ml/g-$SiO_2$ in order to adjust the sodium (Na) content, and then filtered and washed using demineralized water heated to 80°C until the pH of the wash liquid reached 5 or more. The resulting slurry was dried with a spray dryer (Mini Spray Dryer B290, manufactured by Nippon BUCHI K.K.) to obtain a silica precursor.

The obtained silica precursor was placed in an alumina crucible and subjected to a heat treatment in an electric furnace at 1200°C for 1 hour. In order to know thermal history during the heat treatment, a referthermo (a molded ceramic powder that shrinks precisely in response to the thermal history) was placed in the same electric furnace. A temperature indicated by the referthermo was 1200°C.

The silica particles after the heat treatment were cooled to room temperature, and the coarsely aggregated silica particles were passed through a stainless steel sieve with an opening size of 150 $\mu$m while being crushed by a rubber spatula, and undersize particles were collected, thereby obtaining a silica powder of Example 1, which is an aggregate of silica particles.

(Example 2)

A silica powder was obtained in the same manner as in Example 1, except that the rotation speed of the homogenizer was changed to 10000 rpm.

(Example 3)

A silica powder was obtained in the same manner as in Example 1, except that the rotation speed of the homogenizer was changed to 5000 rpm.

(Example 4)

A silica powder was obtained in the same manner as in Example 1, except that the heat treatment temperature was changed to 1165°C.

(Example 5)

A silica powder was obtained in the same manner as in Example 1, except that the heat treatment temperature was changed to 1090°C.

(Example 6)

The silica powder obtained in Example 1 and the other silica powder (Sunsphere NP-30, manufactured by AGC Si-Tech Co., Ltd.) were mixed in a mass ratio of 1:1 to obtain a silica powder.

(Example 7)

As the silica powder, Sunsphere NP-20 manufactured by AGC Si-Tech Co., Ltd. was prepared.

(Example 8)

As the silica powder, Sunsphere NP-30 manufactured by AGC Si-Tech Co., Ltd. was prepared.

(Example 9)

As the silica powder, Sunsphere NP-100 manufactured by AGC Si-Tech Co., Ltd. was prepared.

(Example 10)

As a nylon powder, Orgasol 2002 EXD NAT COS manufactured by Arkema K.K. was prepared.
As a result of performing powder X-ray diffraction measurement on the silica powder of Examples 1 to 9, it was found that all Examples were constituted by amorphous silica particles.
The following measurements were performed on the silica powders of Examples 1 to 9 and the nylon powder of Example 10. Results are shown in Table 1.

<Average Circularity of Particles>

A circularity can be determined by photographing a powder with a scanning electron microscope (JCM-7000 manufactured by JEOL Ltd.), obtaining an area and a perimeter of the particle using image analysis software (image analysis software attached to a particle image analyzer "Morphologi4" manufactured by Malvern Panalytical Ltd), and applying the results to the following formula for calculation. An average value of circularity of 20 particles was determined as an average circularity.

$$\text{Circularity} = \text{perimeter of circle with equal projected area/perimeter of particle}$$

Perimeter of circle with equal projected area: observing a particle from directly above, obtaining an area of a shadow of the particle reflected on a plane below, and calculating a circle having an area equal to this area, so as to obtain a length of an outline of the circle
Perimeter of particle: observing the particle from directly above to obtain a length of an outline of the shadow of the particle reflected on the plane below

<50% particle diameter ($D_{50}$), and ratio ($D_{90}/D_{10}$) of 90% particle diameter ($D_{90}$) to 10% particle diameter ($D_{10}$)>

Distilled water was added to the powder to prepare a 0.05 mass% slurry, followed by subjecting to treatment with an ultrasonic cleaner at a frequency of 45 kHz for 2 minutes, and a particle size distribution measuring device using an electrical detection method (Multisizer 4e manufactured by Beckman Coulter, Inc.) was used to calculate a 50% particle diameter in volume-based cumulative particle size distribution ($D_{50}$) (settings: aperture diameter: 50 $\mu$m, effective N number: 50,000, current: 800 $\mu$A, gain: 4, stirring speed: 15, electrolyte: isoton).
Using the same procedure as above, the 90% particle diameter ($D_{90}$) and the 10% particle diameter ($D_{10}$) in the volume-based cumulative particle size distribution were calculated, and a ratio of $D_{90}$ to $D_{10}$ ($D_{90}/D_{10}$) was calculated from the obtained values.

<Specific Surface Area of Powder>

The powder was dried under reduced pressure at 230°C to completely remove water, thereby obtaining a sample. Regarding this sample, the specific surface area was obtained by a multi-point BET method using a nitrogen gas in "TriStar II", which is an automatic specific surface area and pore distribution measuring device manufactured by Micromeritics Instrument Corporation.

<Shearing cohesion of Powder>

The shearing cohesion was measured using a powder bed shear force measuring device (model NS-S500, manufactured by Nano Seeds Corporation) in accordance with JIS Z 8835:2016 under the following conditions, and a yield locus (YL) was obtained by plotting a shear stress (y-axis) corresponding to each normal stress (x-axis). The shearing cohesion was calculated from an intersection of YL and the y-axis.
Conditions: cell inner diameter 43 mm, indentation gap 0.2 mm, indentation speed 0.2 mm/sec, stress relaxation time 100 sec, shear rate 10 $\mu$m/sec, normal load 50 N

<Stress Relaxation Rate of Powder>

Using a powder bed shear force measuring device (model NS-S500, manufactured by Nano Seeds Corporation), a normal load applied to a powder bed was set to 50 N, and the stress relaxation rate (%) corresponding to the normal stress at the start of shearing was calculated using the following formula from a maximum normal load a on an upper surface of the powder bed during pre-consolidation and a normal load b on the upper surface of the powder bed at 100 seconds after stress relaxation.

$$\text{Stress relaxation rate } (\%) = (1 - b/a) \times 100$$

<Aerated Bulk Density, Packed Bulk Density, Degree of Aggregation, Angle of Repose, Angle of Rupture, Angle of Difference, and Degree of Dispersion of Powder>

The measurements were performed using a powder tester (model: PT-X) manufactured by Hosokawa Micron Corporation.

(Aerated Bulk Density)

The powder was gently poured into a special cup having an internal volume of 100 mL, and the cup was filled with powder until the powder overflowed. The powder that overflowed out of the cup was scraped off with an attached blade, and a mass including a mass of the cup was measured. A mass of the powder was calculated by subtracting a mass of the cup, which was measured in advance, from the above value, and the mass was divided by the internal volume of the cup to obtain the aerated bulk density.

(Packed Bulk Density)

After the measurement of the aerated bulk density, a cap was put on the special cup, and then the cup was placed in a tapping holder of a powder tester, and the cup was then further filled with the powder, and a cap cover was attached on the cap. The cup was then tapped 180 times. After tapping was completed, the cap and the cap cover were removed, followed by scraping off the surface of the cup with the attached blade, and a mass including the cup was measured. A mass of the silica powder was calculated by subtracting the mass of the cup, which was measured in advance, from the above value, and the mass was divided by the internal volume of the cup to obtain the packed bulk density.

(Degree of Compression)

From the values of the packed bulk density and aerated bulk density, the degree of compression was measured according to the following formula.

$$\text{Degree of compression } (\%) = \{(\text{packed bulk density - aerated bulk density})/\text{packed bulk density}\} \times 100$$

(Degree of Aggregation)

A sieve with an opening size of 250 $\mu$m, a sieve with an opening size of 150 $\mu$m, and a sieve with an opening size of 75 $\mu$m were set on a vibration table of the powder tester from top to bottom, 2.0 g of powder was placed on the top sieve (with an opening size of 250 $\mu$m) and vibrated with a vibration amplitude of 1.0 mm for 90 seconds, a mass of powder remaining on each sieve was measured, and the degree of aggregation was calculated based on the following formula.

$$\text{Degree of aggregation } (\%) = \{(A + B \times 3/5 + C \times 1/5)/2\} \times 100$$

A: mass of powder on top sieve, B: mass of powder on middle sieve, C: mass of powder on bottom sieve

(Angle of Repose)

A measuring platform with a diameter of 80 mm was placed horizontally, and a sieve with an opening size of 1.7 mm and a funnel (made of stainless steel, upper opening diameter: 70 mm, lower opening diameter: 7 mm, inclination angle: 63 degrees, height from the measuring platform to the lower opening of the funnel: 7.5 cm) were placed above the measuring platform. To the sieve, 250 mL of the powder was gently placed, and then vibrated with an amplitude of 1.5 mm to allow the powder to fall through the sieve and funnel onto the measuring platform, depositing the powder in a cone shape. Next, the cone shape was photographed from directly beside, and an angle between the generating line and the horizontal plane of the cone was measured. A numerical average value of one measurement was taken as the angle of repose.

(Angle of Rupture)

The angle of rupture was measured by applying the same impact three times to the powder for which the angle of repose was measured using an automatic shocker attached to the device, then photographing the conical shape of the sample from directly beside, and measuring an angle between the generating line and the horizontal plane of the cone. A numerical average value of one measurement was taken as the angle of rupture.

(Angle of Difference)

An angle of difference between the angle of repose and the angle of rupture was calculated.

(Degree of Dispersion)

On a top of a degree of dispersion measuring unit, 10 g of a powder was placed, and the powder was allowed to drop onto a watch glass with a diameter of 100 mm placed 60 cm below the degree of dispersion measuring unit. The degree of dispersion (%) was calculated from the mass of the powder accumulated on the watch glass using the following formula.

Degree of dispersion (%) = {(mass (g) of powder before dropping - mass (g) of powder accumulated on watch glass)/ mass (g) of powder before dropping} $\times$ 100

<Evaluation of Powder Friction Properties>

(Static Friction Coefficient)

A static and dynamic friction measuring device (for example, model TL201 Tt manufactured by Trinity-Lab. Inc.) was used, a urethane artificial finger was used as a contactor, and a load was set to 30 gf, a scanning distance was set to 40 mm, and a scanning speed was set to 10 mm/sec, and artificial leather "sapurare" (manufactured by IDEATEX JAPAN Co., Ltd.) was used as a coating substrate, and the friction coefficient was measured with a coating amount of the powder being 0.8 $\mu$L/cm$^2$ in terms of a bulk volume per unit area. A maximum value of obtained friction coefficients in a range of 0 msec to 1,000 msec was regarded as the static friction coefficient.

(Dynamic Friction Coefficient)

A static and dynamic friction measuring device (for example, model TL201 Tt manufactured by Trinity-Lab. Inc.) was used, the friction coefficient was measured in the same manner as above, and an average value of the obtained friction coefficients in a range of 1,000 msec to 4,000 msec was regarded as the dynamic friction coefficient.

<Texture Evaluation>

(Powder: Examples 1 to 10)

The texture of the powders of Examples 1 to 10 obtained above was evaluated by five panelists in a sensory manner according to the following procedure. Evaluation results are shown in Table 1.

(Procedure)

An appropriate amount of powder (approximately one spoonful with a micro spatula) was placed on the back of a hand or the inside of forearm and rubbed in with fingers. At this time, the "moist feel" and "softness" were evaluated according to the following criteria. An average score for each item was calculated and the texture was evaluated.

[Moist Feel]

5 points: moist
1 point: dry

[Softness]

5 points: soft
1 point: hard

[Table 1]

|  |  |  | Example 1 | Example 2 | Example 3 | Example 4 | Example 5 |
|---|---|---|---|---|---|---|---|
| Physical property | Average circularity | - | 0.976 | 0.982 | 0.968 | 0.962 | 0.971 |
| | $D_{10}$ | μm | 1.9 | 2.5 | 4.9 | 1.9 | 1.8 |
| | $D_{50}$ | μm | 3.7 | 4.1 | 9.7 | 3.6 | 3.7 |
| | $D_{90}$ | μm | 4.2 | 7.0 | 15.7 | 4.3 | 4.2 |
| | $D_{90}/D_{10}$ | - | 2.2 | 2.8 | 3.2 | 2.3 | 2.3 |
| | Specific surface area | $m^2/g$ | 1.2 | 0.8 | 0.4 | 2.3 | 4.0 |
| Powder bed shear test | Shearing cohesion (50 N) | kPa | 3.6 | 2.9 | 2.6 | 3.3 | 2.8 |
| | Stress relaxation rate (50 N) | % | 14 | 11 | 9 | 12 | 11 |
| Powder tester | Aerated bulk density | $g/cm^3$ | 0.51 | 0.55 | 0.75 | 0.53 | 0.53 |
| | Packed bulk density | $g/cm^3$ | 1.09 | 1.12 | 1.24 | 1.07 | 1.08 |
| | Degree of compression | % | 53.3 | 50.8 | 39.9 | 52.6 | 51.4 |
| | Degree of aggregation | % | 100 | 99.2 | 98 | 99.4 | 99.6 |
| | Angle of repose | deg (°) | 42 | 32 | 41 | 39 | 41 |
| | Angle of rupture | deg (°) | 33 | 31 | 33 | 32 | 33 |
| | Angle of difference | deg (°) | 9 | 1 | 8 | 7 | 8 |
| | Degree of dispersion | % | 3.9 | 3 | 11 | 4.1 | 5.2 |
| Friction property | Static friction coefficient | - | 0.89 | 0.87 | 0.54 | 0.75 | 0.67 |
| | Dynamic friction coefficient | - | 0.7 | 0.63 | 0.38 | 0.56 | 0.49 |

(continued)

| | | | Example 1 | Example 2 | Example 3 | Example 4 | Example 5 |
|---|---|---|---|---|---|---|---|
| Texture evaluation | Moist feel | - | 4.6 | 4.2 | 3.4 | 4.4 | 3.8 |
| | Softness | - | 4.6 | 4.2 | 3.2 | 4.2 | 3.8 |

[Table 1] (continued)

| | | | Example 6 | Example 7 | Example 8 | Example 9 | Example 10 |
|---|---|---|---|---|---|---|---|
| Physical property | Average circularity | - | 0.966 | 0.973 | 0.957 | 0.967 | 0.735 |
| | $D_{10}$ | $\mu$m | 2.3 | 1.7 | 2.6 | 5.2 | - |
| | $D_{50}$ | $\mu$m | 3.9 | 3.1 | 4.4 | 9.6 | 11.7 |
| | $D_{90}$ | $\mu$m | 5.7 | 3.9 | 7.2 | 16.3 | - |
| | $D_{90}/D_{10}$ | - | 2.5 | 2.3 | 2.8 | 3.1 | - |
| | Specific surface area | $m^2$/g | 16 | 60 | 32 | 46 | - |
| Powder bed shear test | Shearing cohesion (50 N) | kPa | 2.9 | 1.9 | 1.8 | 0.9 | 2.6 |
| | Stress relaxation rate (50 N) | % | 26 | 36 | 31 | 19 | 30 |
| Powder tester | Aerated bulk density | g/cm$^3$ | 0.59 | 0.59 | 0.59 | 0.81 | 0.3 |
| | Packed bulk density | g/cm$^3$ | 0.98 | 0.89 | 0.94 | 1.16 | 0.5 |
| | Degree of compression | % | 40.3 | 33.7 | 36.9 | 30.7 | 39.2 |
| | Degree of aggregation | % | 67.4 | 50 | 45.5 | 20.6 | 58.1 |
| | Angle of repose | deg (°) | 47 | 44 | 32 | 44 | 46 |
| | Angle of rupture | deg (°) | 27 | 26 | 16 | 16 | 31 |
| | Angle of difference | deg (°) | 20 | 18 | 16 | 28 | 15 |
| | Degree of dispersion | % | 24.1 | 65.7 | 58.8 | 55.5 | 28.4 |
| Friction property | Static friction coefficient | - | 0.46 | 0.42 | 0.42 | 0.46 | 0.49 |
| | Dynamic friction coefficient | - | 0.34 | 0.25 | 0.23 | 0.19 | 0.25 |
| Texture evaluation | Moist feel | - | 3.2 | 2.2 | 1.4 | 1.0 | 4.2 |
| | Softness | - | 3.2 | 2.2 | 2.4 | 1.2 | 4.2 |

From the results shown in Table 1, the silica powders of Examples 1 to 6, which are the working examples, exhibited superior moist feel and softness as compared with Comparative Examples. On the other hand, the silica powders of Examples 7 to 9, which are Comparative Examples, had the degree of aggregation of less than 60%, the angle of difference between the angle of repose and the angle of rupture of more than 15 degrees, and the degree of dispersion of more than 50%, so that the texture related to the moist feel and softness was inferior.

<Cosmetic Material>

(Powder Foundation: Examples 11 to 18)

Powder foundations were prepared according to compositions shown in Table 2 below.

(Production Method)

1) Group A was weighed and mixed uniformly in a Henschel mixer (HANIL ELECTRIC CO., LTD., Hanil Mixer, Model LM-110T) three times for 30 seconds.

2) Group B was weighed and mixed uniformly with a spatula.

3) Group B was added to Group A which had been mixed uniformly, and mixed three times for 30 seconds in a Henschel mixer until uniform, to obtain a powder foundation.

[Table 2]

(Mixing ratio: mass%)

| | | Example 11 | Example 12 | Example 13 | Example 14 | Example 15 | Example 16 | Example 17 | Example 18 |
|---|---|---|---|---|---|---|---|---|---|
| A | Magnesium stearate-treated talc (*1) | 62.19 | 62.19 | 62.19 | 62.19 | 62.19 | 62.19 | 62.19 | 62.19 |
| | Magnesium stearate-treated mica (*2) | 22.00 | 22.00 | 22.00 | 22.00 | 22.00 | 22.00 | 22.00 | 22.00 |
| | Particles of Example 1 | 10.00 | - | - | - | - | - | - | - |
| | Particles of Example 2 | - | 10.00 | - | - | - | - | - | - |
| | Particles of Example 5 | - | - | 10.00 | - | - | - | - | - |
| | Particles of Example 6 | - | - | - | 10.00 | - | - | - | - |
| | Particles of Example 7 | - | - | - | - | 10.00 | - | - | - |
| | Particles of Example 8 | - | - | - | - | - | 10.00 | - | - |
| | Particles of Example 9 | - | - | - | - | - | - | 10.00 | - |
| | Particles of Example 10 | - | - | - | - | - | - | - | 10.00 |
| | Magnesium stearate-treated titanium dioxide (*3) | 3.00 | 3.00 | 3.00 | 3.00 | 3.00 | 3.00 | 3.00 | 3.00 |
| | Yellow iron oxide (*4) | 0.30 | 0.30 | 0.30 | 0.30 | 0.30 | 0.30 | 0.30 | 0.30 |
| | Red iron oxide (*5) | 0.10 | 0.10 | 0.10 | 0.10 | 0.10 | 0.10 | 0.10 | 0.10 |
| | Black iron oxide (*6) | 0.01 | 0.01 | 0.01 | 0.01 | 0.01 | 0.01 | 0.01 | 0.01 |
| B | Mixture of diheptyl succinate and (capryloyl glycerin/sebacic acid) copolymer (*7) | 2.00 | 2.00 | 2.00 | 2.00 | 2.00 | 2.00 | 2.00 | 2.00 |
| | Mixture of ethylhexylglycerin and glyceryl caprylate (*8) | 0.40 | 0.40 | 0.40 | 0.40 | 0.40 | 0.40 | 0.40 | 0.40 |
| | Total | 100 | 100 | 100 | 100 | 100 | 100 | 100 | 100 |
| Texture | Moist feel | 4.8 | 4.4 | 4.2 | 4.2 | 3.0 | 2.8 | 2.2 | 4.2 |

EP 4 538 230 A1

(continued)

(Mixing ratio: mass%)

| | | Example 11 | Example 12 | Example 13 | Example 14 | Example 15 | Example 16 | Example 17 | Example 18 |
|---|---|---|---|---|---|---|---|---|---|
| evaluation | Softness | 4.4 | 4.4 | 3.8 | 4.2 | 3.6 | 3.4 | 2.2 | 4.4 |

In Table 2, (*1) to (*8) are as follows.
(*1) Magnesium stearate-treated talc: MST-1 TALC SX (Daito Chemical Industry Co., Ltd.)
(*2) Magnesium stearate-treated mica: MST-2 SERICITE S (Daito Chemical Industry Co., Ltd.)
(*3) Magnesium stearate-treated titanium dioxide: MST-1 TiO2 R250 (Daito Chemical Industry Co., Ltd.)
(*4) Yellow iron oxide: SunPURO Yellow Iron Oxide: (SUNCHEMICAL)
(*5) Red iron oxide: SunPURO Red Iron Oxide
(*6) Black iron oxide: SunPURO Red Iron Oxide
(*7) Mixture of diheptyl succinate and (capryloyl glycerin/sebacic acid) copolymer
(*8) Mixture of ethylhexylglycerin and glyceryl caprylate: NIKKOGUARD

The texture of each of the powder foundations obtained above was evaluated by five panelists in a sensory manner according to the following procedure.

[Procedure]

An appropriate amount of the powder foundation (approximately one spoonful with a micro spatula) was placed on the back of a hand or the inside of forearm and rubbed in with fingers. At this time, the "moist feel", "softness" and "spreadability" were evaluated according to the following criteria. An average score for each item was calculated and the texture was evaluated.

[Moist Feel]

5 points: moist
1 point: dry

[Softness]

5 points: soft
1 point: hard

In terms of the "moist feel", "softness" and "spreadability", the powder foundations of Examples 11 to 14 had a texture closer to that of the powder foundation of Example 18 than Examples 15 to 17.
In order to impart similar effects, the silica powder of the present embodiment can also be mixed into other preparations (including emulsion-based preparations), such as pressed foundations, face colors (eye shadows, blushers), face powders, highlighters, eyebrow products, dry shampoos, bath additives, and powder deodorants.

(Water-based Gel: Examples 19 to 21)

Water-based gels were prepared according to compositions shown in Table 3 below.

(Production Method)

1) Previously dispersed Group B was added to Group A, followed by stirring until uniform to obtain a first composition.
2) While stirring the first composition, a Group C aqueous solution was added, stirring was continued until the mixture became uniform, to obtain a second composition.
3) Group D was added to the second composition, followed by stirring until uniform to obtain a third composition.
4) Water or silica dispersion of Group E was added to the third composition, followed by stirring until uniform to obtain a water-based gel.

[Table 3]

| | | | (Mixing ratio: mass%) | | |
|---|---|---|---|---|---|
| | | Components | Example 19 | Example 20 | Example 21 |
| A | | Water | 67.7 | 67.7 | 67.7 |
| | | Propanediol (*1) | 10.0 | 10.0 | 10.0 |
| B | | Water | 14.7 | 14.7 | 14.7 |
| | | Carbomer (*2) | 0.3 | 0.3 | 0.3 |

(continued)

| | | | Example 19 | Example 20 | Example 21 |
|---|---|---|---|---|---|
| | | | | | (Mixing ratio: mass%) |
| | | Components | Example 19 | Example 20 | Example 21 |
| C | | Water | 0.9 | 0.9 | 0.9 |
| | | Hydroxide K (*3) | 0.1 | 0.1 | 0.1 |
| D | | Phenoxyethanol (*4) | 0.3 | 0.3 | 0.3 |
| E | | Water | 6.0 | 3.0 | 3.0 |
| | | Particles of Example 2 | - | 3.0 | - |
| | | Particles of Example 8 | - | - | 3.0 |

In Table 3, (*1) to (*4) are as follows.
(*1) Propanediol: ZEMEA Select Propanediol (DuPont Tate&Lyle Bio Products)
(*2) Carbomer: Carbopol Ultrez 10 polymer (Lubrizol)
(*3) Hydroxide K: Potassium hydroxide (Kanto Chemical Co., Inc.)
(*4) Phenoxyethanol: Phenoxyethanol-S (Yokkaichi Chemical Company Limited)

The water-based gels of Examples 19 to 21 obtained above were subjected to a sensory evaluation by one panelist according to the following procedure.

[Procedure]

An appropriate amount of the water-based gel (approximately one spoonful with a micro spatula) was applied to the back of the hand and rubbed in with the fingertips.
As a result of the sensory evaluation, Example 20 provided a better texture than Examples 19 and 21.
In order to impart similar effects, the silica powder of the present embodiment can also be mixed into other preparations (including emulsion-based preparations), such as lotions, beauty serums, masks, peel-off type packs, brush-type eyeliners and eyebrow products, hair growth agents, repellents, milk lotions, gel creams, face creams, hand creams, body creams, sunscreens, makeup bases, foundations, concealers, mascaras, cleansing milks, cleansing creams, rinse-off packs, enamel treatments, rinses, hair treatments, color treatments, hair styling products, and deodorants.

(Lipstick: Examples 22 to 24)

Lipsticks were prepared according to compositions shown in Table 4 below.

(Production Method)

1) Group A was weighed and heated at 120°C, and stirred in a dispersing mixer until uniform to obtain a first composition.
2) Group B was added to the first composition, and stirring was continued with a dispersing mixer until the mixture became uniform, to obtain a second composition.
3) Group C was added to the second composition and , followed by stirring until uniform to obtain a third composition.
4) The third composition was poured into a lipstick container and allowed to stand at room temperature for 5 minutes and at -15°C for 15 minutes to obtain a lipstick.

[Table 4]

(Mixing ratio: mass%)

| | Components | Example 22 | Example 23 | Example 24 |
|---|---|---|---|---|
| A | Diisostearyl malate (*1) | 13.0 | 13.0 | 14.0 |
| | Hydrogenated polyisobutene (*2) | 12.5 | 12.5 | 13.5 |
| | Paraffine (*3) | 12.0 | 12.0 | 12.5 |
| | Alkyl benzoate (C12-15) (*4) | 10.0 | 10.0 | 11.0 |
| | Mixture of diheptyl succinate and (capryloyl glycerin/sebacic acid) copolymer (*5) | 9.0 | 9.0 | 9.0 |
| | Octyldodecanol (*6) | 6.0 | 6.0 | 6.5 |
| | Diisopropyl dilinoleate (*7) | 6.0 | 6.0 | 6.5 |
| | Pentaerythrityl tetraethylhexanoate (*8) | 6.0 | 6.0 | 6.0 |
| | Pentaerythrityl tetraisostearate (*9) | 6.0 | 6.0 | 6.0 |
| | Polyglyceryl-2 isostearate (*10) | 4.5 | 4.5 | 4.5 |
| | Synthetic wax (*11) | 2.5 | 2.5 | 3.0 |
| | Microcrystalline wax (*12) | 1.8 | 1.8 | 1.8 |
| | Propylparaben (*13) | 0.1 | 0.1 | 0.1 |
| | Tocopherol (*14) | 0.1 | 0.1 | 0.1 |
| B | White pigment paste (titanium oxide, polyglyceryl-2 triisostearate) (*15) | 1.0 | 1.0 | 1.0 |
| | Red pigment paste (polyglyceryl-2 triisostearate, Red 202) (*16) | 1.0 | 1.0 | 1.0 |
| | Red pigment paste (Red 226, polyelyceryl-2 triisostearate) (*17) | 1.2 | 1.2 | 1.2 |
| | Yellow pigment paste (polyglyceryl-2 triisostearate, Yellow 4) (*18) | 0.3 | 0.3 | 0.3 |

(continued)

| | Components | Example 22 | Example 23 | Example 24 |
|---|---|---|---|---|
| | | (Mixing ratio: mass%) | | |
| C | Pearl pigment (titanium oxide-coated mica) (*19) | 2.0 | 2.0 | 2.0 |
| | Particles of Example 2 | 5.0 | - | - |
| | Particles of Example 8 | - | 5.0 | - |

In Table 4, (*1) to (*19) are as follows.
(*1) Diisostearyl malate: Cosmol 222 (Nisshin Oillio Group, Ltd.)
(*2) Hydrogenated polyisobutene: PARLEAM 18 (NOF Corporation)
(*3) Paraffine: Paraffin Wax-125 (NIPPON SEIRO Co., Ltd.)
(*4) Alkyl benzoate (C12-15): Crodamol AB (Croda Japan K.K.)
(*5) Mixture of diheptyl succinate and (capryloyl glycerin/sebacic acid) copolymer: LexFeel N5 MB (INOLEX, INC.)
(*6) Octyldodecanol: RISONOL 20SP (KOKYU ALCOHOL KOGYO CO., LTD.)
(*7) Diisopropyl dilinoleate: KAK DADIP-R (KOKYU ALCOHOL KOGYO CO., LTD.)
(*8) Pentaerythrityl tetraethylhexanoate: SALACOS 5408 (Nisshin Oillio Group, Ltd.)
(*9) Pentaerythrityl tetraisostearate: SALACOS 5418V (Nisshin Oillio Group, Ltd.)
(*10) Polyglyceryl-2 isostearate: Cosmol 41V (Nisshin Oillio Group, Ltd.)
(*11) Synthetic wax: Microease 114S (MICRO Powders, INC.)
(*12) Microcrystalline wax: MULTIWAX W-445 (Sonneborn LLC)
(*13) Propylparaben: MEKKINS-P (C) (UENO FINE CHEMICALS INDUSTRY, LTD.)
(*14) Tocopherol: Tocopherol 100 (Nisshin Oillio Group, Ltd.)
(*15) White pigment paste (titanium oxide, polyglyceryl-2 triisostearate): DK-PGT PASTE Ti (Daito Kasei Kogyo Co., Ltd.)
(*16) Red pigment paste (polyglyceryl-2 triisostearate, Red 202): DK-PGT PASTE R7 (Daito Kasei Kogyo Co., Ltd.)
(*17) Red pigment paste (Red 226, polyglyceryl-2 triisostearate): a mixture in which DK D&C RED No. 30 was dispersed at 30 wt% into Cosmol 43V (Nisshin Oillio Group Co., Ltd.)using a three-roll mill (EXAKT, EXAKT Technologies, Inc.)
(*18) Yellow pigment paste (polyglyceryl-2 triisostearate, Yellow 4): DK-PGT PASTE Y5L (Daito Kasei Kogyo Co., Ltd.)
(*19) Pearl pigment: Sunpuro Fine white (SunChemical Corporation)

The lipsticks of Examples 22 to 24 obtained above were subjected to a sensory evaluation by one panelist according to the following procedure.

[Procedure]

The lipstick was applied to lips. At this time, spreadability was evaluated.
As a result of the sensory evaluation, the lipsticks of Examples 22 and 23 were superior to Example 24 in terms of spreadability.
In order to impart similar effects, the silica powder of the present embodiment can also be mixed into other solid oil-based preparations, such as foundations, concealers, lip balms, eyeliners, eyebrow products, stick deodorants, stick eyeshadows, stick beauty serums, stick highlighters, cleansing balms, and hair styling products.
Although various embodiments have been described above, it is needless to say that the present invention is not limited to such examples. It is obvious for a person skilled in the art that various modifications and variations can be made within the category described in the scope of claims and it is understood that such modifications and variations naturally belong to the technical scope of the present invention. Further, the components described in the above embodiments may be combined in any manner without departing from the spirit of the invention.
Note that, the present application is based on Japanese Patent Application No. 2022-095198 filed on June 13, 2022 and Japanese Patent Application No. 2023-006655 filed on January 19, 2023, the contents of which are incorporated herein by reference.

**Claims**

1. A silica powder for a cosmetic material, comprising:

amorphous silica particles, wherein
the silica particles have an average circularity of 0.75 or more, and
the silica powder satisfies at least one of the following conditions (1) to (3),

(1) a degree of aggregation of 60% or more,
(2) an angle of difference between an angle of repose and an angle of rupture of 15 degrees or less, or
(3) a degree of dispersion of 50% or less.

2. The silica powder for a cosmetic material according to claim 1, having a stress relaxation rate of 18% or less when a load of 50 N is applied.

3. The silica powder for a cosmetic material according to claim 1 or 2, having a shearing cohesion of 2.0 kPa or more when a load of 50 N is applied.

4. The silica powder for a cosmetic material according to claim 1 or 2, having a specific surface area of less than 5 m$^2$/g.

5. The silica powder for a cosmetic material according to claim 1 or 2, wherein

the silica powder has a 50% particle diameter ($D_{50}$) in a volume-based cumulative particle size distribution of 0.5 $\mu$m to 100 $\mu$m, and
the silica powder has a ratio ($D_{90}/D_{10}$), which is a ratio of a 90% particle diameter ($D_{90}$) in the volume-based cumulative particle size distribution to a 10% particle diameter ($D_{10}$) in the volume-based cumulative particle size distribution, of 1.0 to 5.0.

6. A cosmetic material comprising the silica powder for a cosmetic material according to claim 1 or 2.

7. The cosmetic material according to claim 6, further comprising one or more types of silica powders other than the silica powder for a cosmetic material.

## INTERNATIONAL SEARCH REPORT

| | International application No. |
|---|---|
| | **PCT/JP2023/021631** |

**A. CLASSIFICATION OF SUBJECT MATTER**

*C01B 33/193*(2006.01)i; *A61Q 1/12*(2006.01)i; *A61K 8/25*(2006.01)i
FI: A61K8/25; C01B33/193; A61Q1/12

According to International Patent Classification (IPC) or to both national classification and IPC

**B. FIELDS SEARCHED**

Minimum documentation searched (classification system followed by classification symbols)

A61K8/00-8/99; A61Q1/00-90/00; C01B33/12-C01B33/193

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X | JP 4-83712 A (ASAHI GLASS CO LTD) 17 March 1992 (1992-03-17)<br>p. 2, upper left column, line 3 to p. 3, lower left column, line 6 | 1-7 |
| X | JP 2021-6513 A (NIPPON SHOKUBAI CO., LTD.) 21 January 2021 (2021-01-21)<br>paragraphs [0001]-[0061], [0147], [0161], [0168]-[0186] | 1-7 |
| A | JP 2017-57094 A (FUJI XEROX CO LTD) 23 March 2017 (2017-03-23) | 1-7 |
| A | JP 2005-60263 A (DOKAI CHEMICAL INDUSTRIES CO LTD) 10 March 2005 (2005-03-10) | 1-7 |
| A | JP 11-302011 A (DOKAI CHEMICAL INDUSTRIES CO LTD) 02 November 1999 (1999-11-02) | 1-7 |
| A | JP 3-193619 A (NITTO CHEMICAL INDUSTRY CO LTD) 23 August 1991 (1991-08-23) | 1-7 |
| A | WO 2011/049121 A1 (NIPPON SHOKUBAI CO., LTD.) 28 April 2011 (2011-04-28) | 1-7 |
| P, X | JP 2022-102438 A (AGC INC) 07 July 2022 (2022-07-07)<br>paragraphs [0095]-[0116] | 1-6 |

☐ Further documents are listed in the continuation of Box C.  ☑ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **06 July 2023** | **18 July 2023** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

**INTERNATIONAL SEARCH REPORT**
Information on patent family members

International application No.

**PCT/JP2023/021631**

| Patent document cited in search report | | | Publication date (day/month/year) | Patent family member(s) | | | Publication date (day/month/year) |
|---|---|---|---|---|---|---|---|
| JP | 4-83712 | A | 17 March 1992 | (Family: none) | | | |
| JP | 2021-6513 | A | 21 January 2021 | (Family: none) | | | |
| JP | 2017-57094 | A | 23 March 2017 | US | 2017/0073236 | A1 | |
| | | | | CN | 106517214 | A | |
| JP | 2005-60263 | A | 10 March 2005 | (Family: none) | | | |
| JP | 11-302011 | A | 02 November 1999 | (Family: none) | | | |
| JP | 3-193619 | A | 23 August 1991 | (Family: none) | | | |
| WO | 2011/049121 | A1 | 28 April 2011 | US | 2012/0202923 | A1 | |
| | | | | CN | 102574692 | A | |
| | | | | KR | 10-2012-0085290 | A | |
| JP | 2022-102438 | A | 07 July 2022 | (Family: none) | | | |

Form PCT/ISA/210 (patent family annex) (January 2015)

**REFERENCES CITED IN THE DESCRIPTION**

**Patent documents cited in the description**

- JP 2921058 B
- WO 2013062105 A
- JP 2022095198 A
- JP 2023006655 A